(19) Europäisches Patentamt
European Patent Office
Office européen des brevets

(11) EP 2 046 866 B1

(12) EUROPEAN PATENT SPECIFICATION

(45) Date of publication and mention of the grant of the patent:
**10.11.2010 Bulletin 2010/45**

(21) Application number: **07840635.2**

(22) Date of filing: **01.08.2007**

(51) Int Cl.:
***C08G 77/14*** *(2006.01)* ***C08G 77/46*** *(2006.01)*
***A61K 8/89*** *(2006.01)*

(86) International application number:
**PCT/US2007/074929**

(87) International publication number:
**WO 2008/016957 (07.02.2008 Gazette 2008/06)**

# (54) SILOXANE POLYMERS AND USES THEREOF

SILOXANPOLYMERE UND ANWENDUNGEN DAVON

POLYMÈRES DE SILOXANE ET LEURS UTILISATIONS

(84) Designated Contracting States:
**DE ES FR GB IT**

(30) Priority: **02.08.2006 US 835013 P**

(43) Date of publication of application:
**15.04.2009 Bulletin 2009/16**

(73) Proprietor: **Honeywell International Inc.**
**Morristown, NJ 07962 (US)**

(72) Inventor: **ASIRVATHAM, Edward T.**
**Chatham, New Jersey 07928-2266 (US)**

(74) Representative: **Hucker, Charlotte Jane**
**Kilburn & Strode LLP**
**20 Red Lion Street**
**London**
**WC1R 4PJ (GB)**

(56) References cited:
**WO-A-2005/049697** **WO-A-2006/014328**
**WO-A-2006/122707** **US-A- 5 021 601**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 046 866 B1

## Description

### FIELD OF INVENTION

This invention relates generally to siloxane compositions. More specifically, this invention relates to methods of producing siloxane polymers having aldehyde functionality, as well as compositions and uses of the same.

### BACKGROUND

Siloxane compounds constitute an important class of industrial chemicals that are commonly found in organic copolymers of various forms including fluids, gels, elastomers, and resins. By modifying a siloxane with certain organofunctional groups and then linking these compounds to form an organic polymer, compositions can be formed having a wide variety of desirable physical and chemical properties such as improved impact resistance, flame resistance, heat stability, lubricity, and flow properties. Many of these compounds have use in such diverse applications as wetting agents, manufacturing processing aides, surfactants, foam control additives, pressure sensitive adhesives, thermoplastic elastomers, compatibilizing agents, water repellant materials, dry cleaning fluids, textile aids, personal and household care, preservatives, pesticides, and electronic circuits. In addition, many of these polymers and copolymers are non-toxic and environmentally compatible and can effectively be used in cosmetic and personal care products. Examples of modified siloxanes include aryl-functional silicone (see, e.g., US 6,716,441); silicone acrylates (see, e.g., US 6,630,133); amino-, epoxy-, and anhydride-functional silicones (see, e.g., US 2003/0162688); and silicone-fluoroalkyl polyether (see, e.g., JP 2006 045102).

Of particular interest to the present invention is siloxane polymers having aldehyde functionality. Aldehyde functional siloxanes are reactive with several synthetic and natural compounds. For example, aldehyde functional groups can react with sugars, starches, sucrose esters, and cellulose to form acetal derivatives, and with proteins, amino acids, and peptides to form imine derivatives.

Aldehyde functional siloxanes, particularly siloxane polymers having terminal aldehyde functionality, can also be utilized as part of a multifunctional polymer or copolymer by either blending two or more types of reactive polymers or by forming a copolymer comprising the aldehyde functional siloxane. These multifunctional polymers, which can have properties that are not achievable from their individual polymer ingredients alone, are particularly useful in formulating personal care products. For example, siloxane polymers and multifunctional copolymers can suspend biologically and/or cosmetically active ingredients via encapsulation, etc., and deliver these active ingredients to the desired site of activation such as the skin, nails, or hair. Using a siloxane polymer in this way can minimize the concentration of active ingredients of a personal care product (e.g., via time release dosing), thus reducing adverse side effects such as irritation. In addition, such polymers and copolymers provide for rheological control, hydrophobicity, emolliency, pigment dispersion, good film forming properties, lubrication, adhesion, foam control, surface modification, cationic/anionic surfactant, and can also provide a product with a desirable tactile impression such as soft-silky feel. Multifunctional polymers may also provide a more economic means of producing certain personal care products see WO 2005/049 697, e.g., by reducing the number of formulations required for a particular product line.

Aldehyde-functional siloxanes, as well as polymer and copolymers derived therefrom, have been described in WO 2006/014328 and WO 2006/014367, both of which are assigned to the same assignee as the present application.

### SUMMARY OF THE INVENTION

The Applicants have discovered a method of preparing functionalized siloxane wherein a redox initiator having a specific functionality is formed and then reacted with hydride end-capped siloxane to produce a siloxane polymer. Such polymers have a plurality of terminal functional groups, such as aldehydes, which produce better adhesion characteristics compared to bi-functional polymers. The improved adhesion is believed to result from the increased number of reactive sites on the polymer.

In addition, such siloxane polymers having aldehyde functionality can be reacted with other monomer or polymers having different reactive functional groups to form a multifunctional copolymer. Such copolymers can posses functionalities that are not achievable from the individual polymers.

The present invention in its various aspects is as set out in the appended claims.

The present invention provides a siloxane polymer comprising a plurality of moieties having the structure:

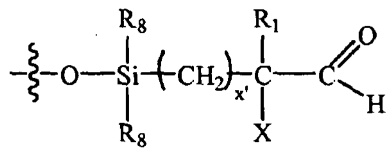

wherein X is hydrogen, chloride, bromide, or iodide; x' is an integer from 2 to 6; $R_1$ is methyl, ethyl, or phenyl; and $R_8$ is independently selected from the group consisting of hydrogen, $C_1$ - $C_{50}$ straight or branched alkyl, $C_3$ - $C_{12}$ substituted or unsubstituted cyclic, $C_1$ - $C_{11}$ heterocyclic, $C_6$ - $C_8$ aryl, $C_6$ - $C_8$ aryloxy, $C_1$ - $C_{12}$ alkoxy, $C_2$ - $C_{12}$ di-alkylamino, $C_1$ - $C_{12}$ alkylthio, $C_1$ - $C_{12}$ fluoroalkyl, $C_1$ - $C_{12}$ epoxy, $C_1$ - $C_6$ acrylic or methacryoxy, $C_6$ - $C_{50}$ polyether, or some combination thereof; with a second monomer to form a copolymer product comprising the siloxane polymer structure described above and a second monomer or polymer having a different structure.

According to another aspect of the invention, provided is a personal care product comprising at least one siloxane polymer according to the present invention.

According to yet another aspect of the invention, a method of delivering an active ingredient to a bodily surface is provided comprising the step of suspending the active ingredient in a siloxane polymer according to the present invention.

## BRIEF DESCRIPTION OF THE DRAWINGS

FIG. 1 is a gel permeation chromatography (GPC) scan of the copolymer product of Example 1;
FIG. 2 is a gel permeation chromatography (GPC) scan of the copolymer product of Example 2;
FIG. 3 is a gel permeation chromatography (GPC) scan of the copolymer product of Example 3; and
FIG. 4 is an NMR graph of the copolymer product of Example 4.

## DETAILED DESCRIPTION OF THE INVENTION

Provided are methods for producing siloxane polymers that can be reacted with other monomers or polymers to form a multifunctional copolymer. The copolymerization site can be customized to efficiently produce a wide variety of block and graft copolymers.

The term "redox initiator", as used herein, refers to a system which effects the radical polymerization of the polymer. Specifically, a redox initiator, when attached to a siloxane, promotes oxidative coupling between the siloxane and one or more vinyl groups of a monomer or polymer to form block or graft siloxane copolymers. This process of oxidative coupling, which is also known in the art as "redox polymerization", generally involves the transfer of electrons between the redox initiator attached to the siloxane and at least one other monomer or polymer during the copolymerization reaction. Without being bound to any particular theory, it is believed that redox initiators accept an electron during a redox reaction, thereby creating a polymeric siloxane radical. This polymeric radical, in turn, reacts with vinylic monomers and/or polymers to form a siloxane-vinyl block or graft copolymer.

Several redox initiators are known in the art. Those suitable comprise (1) a free radical initiator which serves to facilitate reduction of a polymeric siloxane, (2) an abstraction moiety which is removable from the redox initiator providing a pair of free electrons, (3) a tertiary alpha-carbon which functions as a copolymerization site, (4) a stabilizing constituent for controlling the copolymerization kinetics, and (5) a group capable of being hydrosilylated which serves to attach the redox initiator to the siloxane. Generally, redox initiators will be of Formula (II):

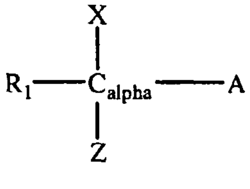

(II)

wherein:

Z is an aldehyde, preferably having carbinol and carboxyl functionalities;
$C_{alpha}$ is the first carbon adjacent to Z;
X is an abstraction moiety;
$R_1$ is a stabilizing constituent; and
A is a group capable of being hydrosilylated, preferably alkene or alkyne.

With respect to the free radical initiator, Z, it is an agent used to start the redox copolymerization reaction involving the polymerizable siloxane. The redox initiator, when attached to the siloxane, must be reducable so as to readily form a polymeric free radical. This polymeric free radical has one unpaired electron that is produced upon the splitting of a molecular bond. That is, the free radical has at least one of the bonding orbitals occupied by a single electron. Once the polymeric radical is formed, it can then undergo oxidative coupling with another monomer and/or polymer. This action starts a chain reaction wherein the radicals that are consumed by the formation of a polymer or copolymer bond are regenerated, thereby leading to the formation of a polymer or copolymer.

With respect to the abstraction moeity, X, it is the moeity that leaves the molecule in order to create the polymeric free radical. Generally, the abstraction site becomes the location of polymeric linkage. These abstraction moieties are a hydrogen or a highly electronegative atom, such as a halogen. Examples of preferred abstraction moieties include, but are not limited to, hydrogen, chlorine, bromine, and iodine.

With respect to the stabilizing constituent, $R_1$, it is a moiety that stabilizes the free radical formed during the polymerization reaction, preferably by resonance forces. It is known that more stable free radicals form more easily. That is, the ease at which radical formation occurs (i.e. the acceptance of an electron and corresponding abstraction of hydrogen or halogen) increases as the stability of the resulting free radical increases. The dissociation energy of the abstraction moeity bond generally provides a measurement of the relative inherent stability of the free radical. With respect to carbon-based free radicals, stability order is as follows:

Tertiary > Secondary > Primary > $CH_4$ > Vinylic

Increasing the number of alkyl substituent on the radical center generally leads to an increase in stability, which is thought to be caused by hyperconjugation. Thus, redox intiators having a radical on a tertiary carbon (i.e. a carbon having only one abstraction moeity) are preferred to redox intiators having a radical on a secondary carbon because the the tertiary carbon-centered radical is more stable due to more distinct resonance stabilization. Such tertiary carbon-centered radicals are formed, for example, by $R_1$ being an alkyl or a phenyl. For the formation of siloxane polymers, preferred stabilizing constituents are methyl, ethyl, and phenyl, with phenyl being particularly preferred.

The stability of free radicals of the present invention is also enhanced by the presence at the radical center of either an electron-donating group or an electron withdrawing group. It is believed that this increased stability arises from the further increase in resonance. Examples of $R_1$ as an electron donating group include, but are not limited to, alkyloxy, aryloxy, thioethers, dialkylamines, or a phenyl substituted, preferably at the fourth carbon, with an alkyoxy, aryloxy, thioether, or dialkylamine. Particularly preferred alkyloxies include those having the formula $-O-R_2$, wherein $R_2$ is a $C_1$ - $C_3$ alkyl. Particular preferred aryloxies include those having the formula $-O-(C_6H_6)$. Particularly preferred thioethers include those having the formula $-S-R_3$, wherein $R_3$ is a $C_1$ - $C_3$ alkyl or a phenyl. Particular preferred dialkylamines include those having the formula $-N(R_4)_2$, wherein $R_4$ is methyl, ethyl, or phenyl. Examples of $R_1$ as an electron withdrawing group include, but are not limited to aryls substituted, preferably at the fourth carbon, with nitro, nitrile, aldehyde, $C_1$ - $C_3$ ketone, or $C_1$ - $C_3$ ester.

The particular $R_1$ substituent incorporated into the redox initiator will depend on the desired reaction kinetics, which can easily be determined by those skilled in the art without undue experimentation. Thus, for copolymerization reactions requiring a decrease in speed and an increase in selectivity, a redox initiator is synthesized having a substituent capable of resonance stabilizing a free radical, such as aromatic rings. In contrast, for copolymerization reactions requiring an increase in speed and a decrease in selectivity, a redox initiator is synthesized with a group having less resonance stabilizing characteristics, such as methyl. The functionality of the $R_1$ substituent may also be considered in choosing a particular $R_1$.

Redox initiators having tertiary carbons are also preferred because polymerization occurs at the site of the hydrogen abstraction and, in the case of tertiary carbons, there is only one abstration site. Restriction of the polymerization reaction to a single site reduces the uncontrollable side-chain reactions and the resulting undesired cross-linked polymers.

With respect to the group capable of being hydrosilylated, A, it is a functional moeity capable of bonding to a siloxane, preferrably via a hydrosilylation reaction, although any chemical process known in the art may be used. Such a hydrosilylation reaction occurs at a silicon-hydrogen bond of a siloxane and involves the addition of the siloxane across the terminal carbon-carbon double bond or triple bond of the redox initiator. Thus, preferably A is an alkene or alkyne,

and more preferably a $C_3$ alkene or alkyne having its double or triple bond, respectively, at a terminal end of the redox initiator distal to the aldehyde group.

Typically, the hydrosilylation process is carried out in the presence of a catalyst, such as platinum. In certain preferred embodiments, the redox initiator bearing the carbon-carbon double or triple bond that can be hydrosilylated attaches to one or both, and more preferably both, ends of the siloxane. Preferred groups capable of being hydrosilylated include vinyl moieties, such as 1-propenyl, 1-butenyl, 1-pentenyl, and the like.

In certain preferred embodiments, the redox initiator of Formula (II) can be further defined wherein:

A is 3-vinyl or 3-allyl;
X is hydrogen or Br;
Z is aldehyde, more preferably methanal, or an aldehyde derived from an acetal such as dimethylacetal; and
$R_1$ is phenyl.

Particularly preferred redox initiators include 2-phenyl-4-pentenal, and 2-phenyl-2-bromo-4-pentenal. Particularly preferred redox initiators wherein Z is an aldehyde derived from an acetal include aldehydes derived from 2-phenyl-(1,1'-dimethoxy)-4-pentene or 2-phenyl-2-bromo-(1,1'-dimethoxy)-4-pentene.

Alternatively, these and other aldehydes can be synthesized by methods known in the art. For example, substituted-4-pentenals can be synthesized as described in U.S. Pat. No. 3,928,644, wherein 2-phenyl-4-pentenal is synthesized from phenyl acetaldehyde.

After the redox initiator is synthesized, it is attached to a siloxane as described above. In certain preferred embodiments, these redox initiators are terminally attached to the siloxane.

As used herein, the term "siloxane" refers to straight-chain compounds having silicon atoms single-bonded to oxygen atoms and so arranged that each silicon atom is linked to at least one oxygen atom. Preferably, siloxanes of the present invention will be silicones (i.e. siloxane polymers based upon a structure consisting of alternating silicon and oxygen atoms with various organic radicals attached to the silicon atoms). In addition, siloxanes suitable for the present invention have at least one silicon-hydrogen bond which serves as the attachment site for the redox initiator. Thus, preferable siloxanes include hydride end-capped siloxanes. Preferably, siloxanes for use with the present invention will be of the following formula (VII):

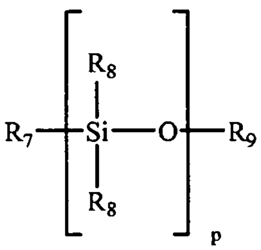

(VII)

wherein $R_7$ is hydrogen;
$R_8$ is independently hydrogen, $C_1$ - $C_{50}$ straight or branched alkyl, $C_3$ - $C_{12}$ substituted or unsubstituted cyclic, $C_1$ - $C_{11}$ heterocyclic, $C_6$ - $C_8$ aryl, $C_6$ - $C_8$ aryloxy, $C_1$ - $C_{12}$ alkoxy, $C_2$ - $C_{12}$ di-alkylamino, $C_1$ - $C_{12}$ alkylthio, $C_1$ - $C_{12}$ fluoroalkyl, $C_1$ - $C_{12}$ epoxy, $C_1$ - $C_6$ acrylic or methacryoxy, $C_6$ - $C_{50}$ polyether, or some combination thereof;
$R_9$ is

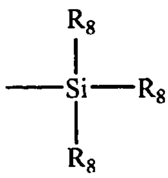

,

provided that at least one $R_8$ constituting the $R_9$ is hydrogen; and
p is an integer from 3 to 40.

Some hydride end-capped siloxanes, mono hydride siloxanes, and rake hydride siloxanes have the following formula:

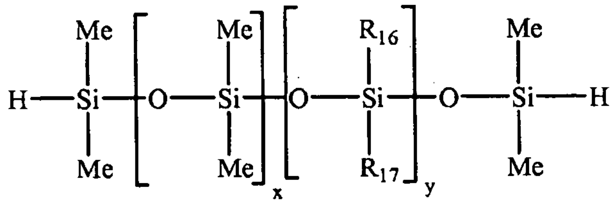

(VIII)

wherein $R_{16}$ and $R_{17}$ are independently methyl or phenyl,
x is an integer from 0 - 80,
y is an integer from 0 - 80, and
x + y 0.

Siloxanes are commercially available from a variety of sources, including for example, dimethylsiloxane - hydrogen terminated (CAS No. 70900-21-9) from Dow Corning, and dimethyl, methylhydrogensiloxane - trimethylsiloxy terminated (CAS No. 68037-59-2) also from Dow Coming.

Alternatively, these and other siloxanes may be prepared by any means known in the art. For example, a polydimethylsiloxane (PDMS) having terminal silicon-hydride functionality may be formed by reacting octamethylcyclotetrasiloxane with dimethyl silane in the presence of $CF_3SO_3H$. A polydimethyl siloxane having pendent silicon-hydride functionality may be formed by reacting octamethylcyclotetrasiloxane and 1,3,5,7-tetramethylcyclotetrasiolxane with tetramethyldisiloxane (TMDS) in the presence of $CF_3SO_3H$.

A preferred method of attaching a redox initiator to the silicon atom is via hydrosilylation. Although not being bound to any particular theory, it is believed that such hydrosilylation reactions occur at a silicon-hydrogen bond of each end of the hydride end-capped siloxane and involve the addition of a siloxane across a carbon-carbon double bond of the redox initiator. Typcially, the hydrosilylation process is carried out in the presence of a catalyst, such as a platinum or platinum-based catalyst.

Once the redox initiator is attached to the siloxane, the compound can participate in a redox copolymerization reaction with vinyl monomers or polymers. The term "polymerizable siloxane", as used herein, refers to siloxane polymers having functional group capable of transforming the siloxane compound into a polymeric radical during a copolymerization process. Typically, the polymerization occurs at the alpha-carbon of the redox initiator derivative (i.e., the carbon adjacent to the aldehyde functional group). Thus, according to another aspect of the present invention, provided are novel siloxane polymers having terminal aldehyde functionality.

It is understood that the polymerizable siloxanes mentioned above are merely exemplary and that many other embodiments of the present invention are also contemplated, including but not limited to, cyclic siloxanes, polycyclic siloxanes, and siloxanes having different functional groups attached to the silicon atoms.

Methods for preparing block and graft copolymers are provided wherein a polymerizable siloxane, such as those described above, is reacted with a vinyl monomer and/or polymer in the presence of a catalyst to produce a siloxane-vinyl copolymer.

As used herein, the term "vinyl" refers to a moiety having, or being derived from, at least the functional group $CH_2$=CH-. The term "vinyl monomer", as used herein, generally refers to vinyl compounds (i.e. compounds having a vinyl functional group), which includes, but is not limited to vinyl chloride, vinyl acetate and similar esters, styrenes, methacrylates, acrylonitriles, and the like. Preferably, the copolymerization reaction involves the formation of a polymeric siloxane radical which is reacted with a vinyl monomer or polymer to yield the siloxane-vinyl copolymer.

A copolymerization process may include the step of mixing aldehyde-functional polymerizable siloxanes with a vinyl monomer and a copper (II) redox catalyst system in a suitable solvent such as benzene, toluene, xylene, glycol, or the like, and heated to 60EC - 125EC for from about 5 to about 24 hours. For preparation of siloxane-polyfluoroolefins, a fluorinated solvent can be used. Once the polymerization reaction is complete, the reaction mixture is cooled to room temperature and mixed with a protic solvent, such as methanol, and the like, to precipitate the copolymer product. The solid product is then washed with a solvent, dried, and purified using typical polymerization techniques known in the art.

The selection of a vinyl monomer for the copolymerization reaction is dependent upon the desired copolymer product. Examples of vinyl monomers that may be used in the present invention include, but are not limited to, ethylene, propylene, styrene, N-vinyl pryrrolidone, vinylidene fluoride, chloroflouoroethylene, methyl methacrylate, ethyl methacrylate, acrylonitrile, hydroxyethyl methacrylate, vinyl acetate, and maleic anhydride. Other examples of vinyl monomers include fluoro olefin monomers such as 3,3,3-trifluoro-1-propene; 2,3,3,3-tetrafluoro-1-propene; 1,3,3,3-tetrafluoro-1-propene; 1-chloro-1,3,3,3-tetrafluoro-1-propene; 2,2,3,3,3-pentafluoro- -propene; 4-vinyl-pyridine.

Depending on the starting materials selected for use in the above-described process, a wide range of novel siloxane-vinyl block and graft copolymers can be efficiently obtained. As used herein, the term "block copolymer" refers to a linear copolymer wherein several monomers of a single first species are proximally connected and then sequentially connected to another chain of proximally connected monomers of another single species that is different than the first species. The term "graft copolymer", as used herein, refers to a non-linear copolymer wherein one or more chains consisting of a single species of monomer are connected to a main polymer chain of a different species as side-chains.

Additionally, by engineering the redox initiator, the copolymerization reactions which occur are more selective thereby leading to a higher yield and better process control of, and less side products in, the desired product stream. As used herein, the term "product stream" refers to a process wherein siloxane and vinyl monomers are reacted to form a product of block or graft copolymers. Although the term "stream" is used, it should be understood that the present invention can be applied to batch or continuous processes. The term "product yield" refers to the weight percentage of targeted copolymer that is formed via a product stream based upon the weight of the reactants.

EXAMPLES

The present invention is further described in light of the following examples which are intended to be illustrative but not limiting in any manner.

Reference examples 1 - 4 demonstrates the preparation of aldehyde functional Silicone-polyethylmethacrylate copolymer.

**Reference Example 1:**

According to this preparation method, a mixture is prepared from the following:

- Methacylic acid ethyl ester (55 mL, 50.4 g, 442 mmol)
- Dry chlorobenzene (140 mL)
- Dry pyridine (7125 μL, 7.0 g)
- Triphenylphosphine (4.2 g)
- Triethylamine (1932 μL, 1.4 g)
- Copper(II)-ethylhexanoate (1.4 g)
- Aldehyde Functional Silicone -AFS 11 (3.5 g, 2.55 mmol)

Methanol for used for workup and chlorobenzene was used for washing purposes. Through the above mentioned mixture a stream of nitrogen is passed for 5 minutes. In an atmosphere of nitrogen, the mixture is heated to 70°C for 21 hours, during which the reaction mixture becomes viscous. After cooling to approximately 40°C, the solution is transferred to a beaker with an additional 50 mL of chlorobenzene. While stirring with an Ultrturrax machine (IKA Laborwerke), methanol (0.8 L) is added. The polymer precipitates as a rod-like material. More methanol (0.6 L) is added while stirring at high speed (14000 rpm). The mother liquor is decanted off. To the viscous polymeric material more methanol (0.5 L) is added while cooling the beaker with ice. The mixture is stirred for approximately 1 hour. The polymeric material is filtered off, washed with methanol, and then dried with air. The process yields 27.5 g of a white product with some lumps. A GPC scan of this product is shown in Figure 1.

**Reference Example 2:**

The procedure of Example 1 was repeated, but without chlorobenzene as a solvent and with the following amounts of reactants and reagents:

- 5.0 mL Methacrylic acid ethyl ester (4.6 g, 40.2 mmol);
- 5.0 mL Catalyst mixture; and
- 150 mg AFS03 (0.42 mol%).

The process yields 3.1 g of isolated product (66 %). A GPC scan of this resulting product is shown in Figure 2.

**Reference Example 3:**

The procedure of Example 1 was repeated, but with the following amounts of reactants and reagents:

- 15 mL styrene; and
- 1.07 g AFS20.

A GPC scan of this resulting product is shown in Figure 3.

**Reference Example 4:**

The procedure of Example 1 was repeated, but with the following amounts of reactants and reagents:

- 10 mL Methacrylic acid ethyl ester (9.17 g, 80.3 mmol);
- 3.21 g AFS11 (2.34 mmol);
- 20 mL chlorobenzene;
- 0.2 g copper(II)-"hexanoate" ;
- 1.0 g pyridine;
- 0.6 g triphenylphosphine; and
- 0.2 g triethylamine.

An NMR scan of the resulting product is shown in Figure 4.

## Claims

**1.** A copolymer comprising a siloxane unit having the structure of:

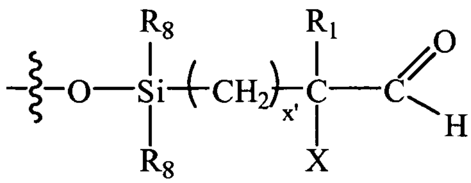

wherein X is hydrogen, chloride, bromide, or iodide;
x' is an integer from 2 to 6;
$R_1$ is methyl, ethyl, or phenyl; and
$R_8$ is independently selected from the group consisting of hydrogen, $C_1$ - $C_{50}$ straight or branched alkyl, $C_3$ - $C_{12}$ substituted or unsubstituted cyclic, $C_1$ - $C_{11}$ heterocyclic, $C_6$ - $C_8$ aryl, $C_6$ - $C_8$ aryloxy, $C_1$ - $C_{12}$ alkoxy, $C_2$ - $C_{12}$ di-alkylamino, $C_1$ - $C_{12}$ alkylthio, $C_1$ - $C_{12}$ fluoroalkyl, $C_1$ - $C_{12}$ epoxy, $C_1$ - $C_6$ acrylic or methacryoxy, $C_6$ - $C_{50}$ polyether, or some combination thereof;

and a second monomer or unit having a different structure selected from the group consisting of sugars, saccharides, and polysaccharides.

**2.** A personal care product comprising a siloxane copolymer according to claim 1.

**3.** The personal care product of claim 2 wherein said copolymer is as a cationic or anionic surfactant, foam control agent, surface modifier, rheology modifier, lubricant, film former, or adhesive.

**4.** A method of delivering a biologically or cosmetically active ingredient to a bodily surface comprising the step of suspending said active ingredient in a siloxane copolymer according to claim 1.

## Patentansprüche

**1.** Copolymer, umfassend eine Siloxaneinheit mit der Struktur:

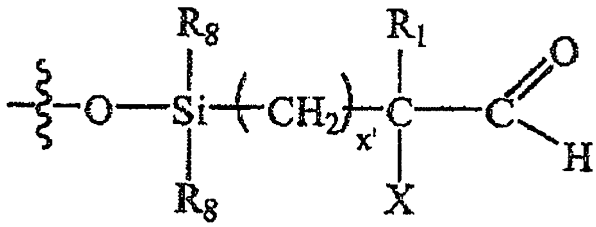

worin X für Wasserstoff, Chlorid, Bromid oder Iodid steht;
x' für eine ganze Zahl von 2 bis 6 steht;
$R_1$ für Methyl, Ethyl oder Phenyl steht und
$R_8$ unabhängig voneinander aus der Gruppe bestehend aus Wasserstoff, geradkettigem oder verzweigtem $C_1$-$C_{50}$-Alkyl, substituiertem oder unsubstituiertem $C_3$-$C_{12}$-Cyclus, $C_1$-$C_{11}$-Heterocyclus, $C_6$-$C_8$-Aryl, $C_6$-$C_8$-Aryloxy, $C_1$-$C_{12}$-Alkoxy, $C_2$-$C_{12}$-Dialkylamino, $C_1$-$C_{12}$-Alkylthio, $C_1$-$C_{12}$-Fluoralkyl, $C_1$-$C_{12}$-Epoxy, $C_1$-$C_6$-Acryl oder -Methacryloxy, $C_6$-$C_{50}$-Polyether oder einer Kombination davon ausgewählt ist;

und ein zweites Monomer oder eine zweite Einheit mit anderer Struktur aus der Gruppe bestehend aus Zuckern, Sacchariden und Polysacchariden.

**2.** Körperpflegeprodukt, umfassend ein Siloxancopolymer gemäß Anspruch 1.

**3.** Körperpflegeprodukt nach Anspruch 2, wobei es sich bei dem Copolymer um ein anionisches oder kationisches Tensid, ein Schaumregulierungsmittel, ein Oberflächenmodifizierungsmittel, ein Rheologiemodifizierungsmittel, ein Gleitmittel, einen Filmbildner oder ein Haftmittel handelt.

**4.** Verfahren zur Zuführung eines biologischen oder kosmetischen Wirkstoffs zu einer Körperoberfläche, bei dem man den Wirkstoff in einem Siloxancopolymer gemäß Anspruch 1 suspendiert.

**Revendications**

**1.** Copolymère comprenant un motif siloxane ayant la structure :

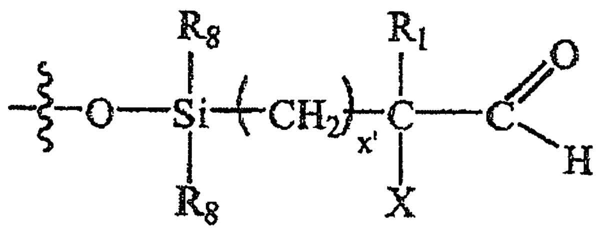

dans laquelle X est hydrogène, chlorure, bromure ou iodure ;
x' est un entier de 2 à 6 ;
$R_1$ est méthyle, éthyle ou phényle ; et
$R_8$ est choisi indépendamment dans le groupe constitué d'hydrogène, alkyle en $C_1$-$C_{50}$ droit ou ramifié, cycle en $C_3$-$C_{12}$ substitué ou non substitué, hétérocycle en $C_1$-$C_{11}$, aryle en $C_6$-$C_8$, aryloxy en $C_6$-$C_8$, alcoxy en $C_1$-$C_{12}$, dialkylamino en $C_2$-$C_{12}$, alkylthio en $C_1$-$C_{12}$, fluoroalkyle en $C_1$-$C_{12}$, époxy en $C_1$-$C_{12}$, méthacryoxy ou acrylique en $C_1$-$C_6$, polyéther en $C_6$-$C_{50}$, ou une combinaison de ceux-ci ;

et un deuxième monomère ou motif ayant une structure différente choisi dans le groupe constitué des sucres, des saccharides et des polysaccharides.

**2.** Produit d'hygiène corporelle comprenant un copolymère de siloxane selon la revendication 1.

3. Produit d'hygiène corporelle de la revendication 2, **caractérisé en ce que** ledit copolymère est un tensioactif cationique ou anionique, un agent de contrôle de mousse, un modifiant de surface, un modifiant de rhéologie, un lubrifiant, un agent filmogène ou un adhésif.

4. Méthode de délivrance sur une surface corporelle d'un ingrédient biologiquement ou cosmétiquement actif comprenant l'étape de mise en suspension dudit ingrédient actif dans un copolymère de siloxane selon la revendication 1.

FIG 1

| | |
|---|---|
| **Kn17/086** | **Methacrylic acid ethyl ester, AFS11** |

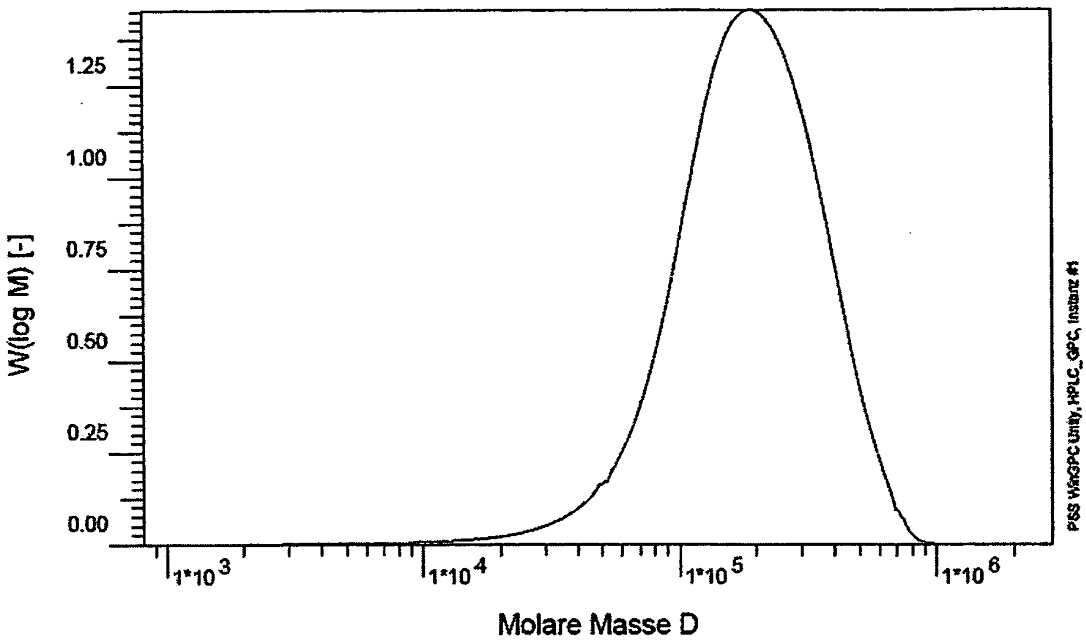

FIG 2

**Analytical Data**

| | |
|---|---|
| Kn16/242 | Methacrylic ethyl ester, AFS03<br>Experiment without chlorobenzene as solvent; mixture got solid |

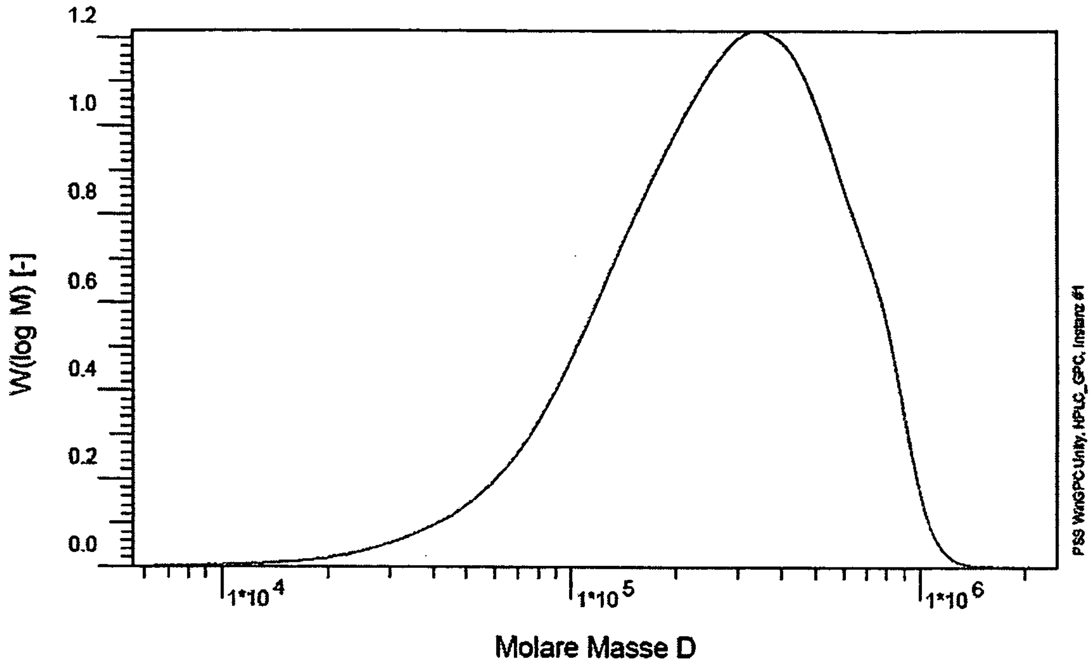

FIG 3

| | |
|---|---|
| **Kn17/103** | **Styrene (15 mL), AFS20 (1.07 g)<br>> 0.5 g left** |

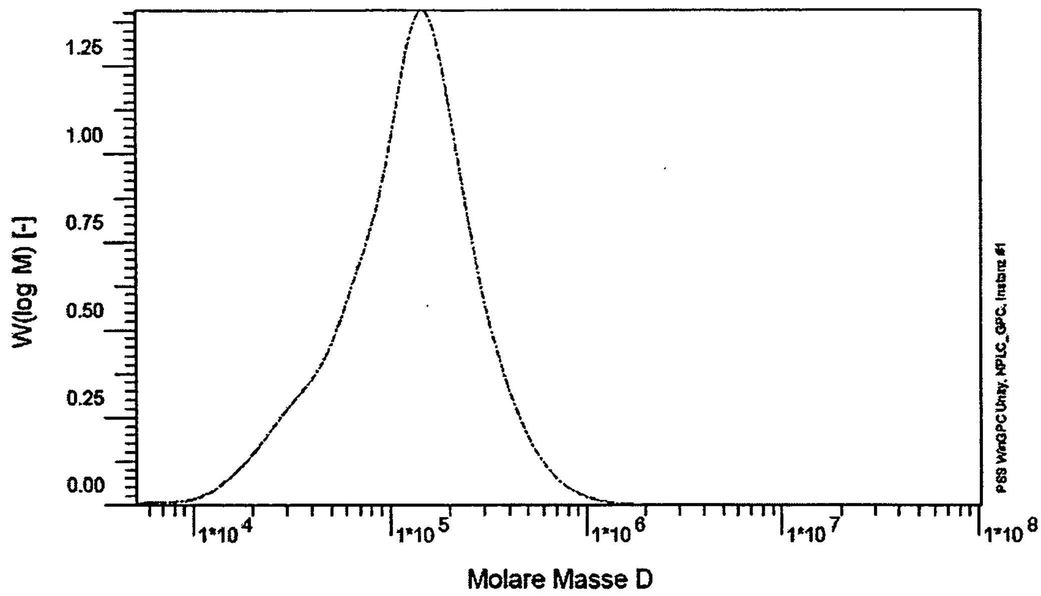

FIG 4

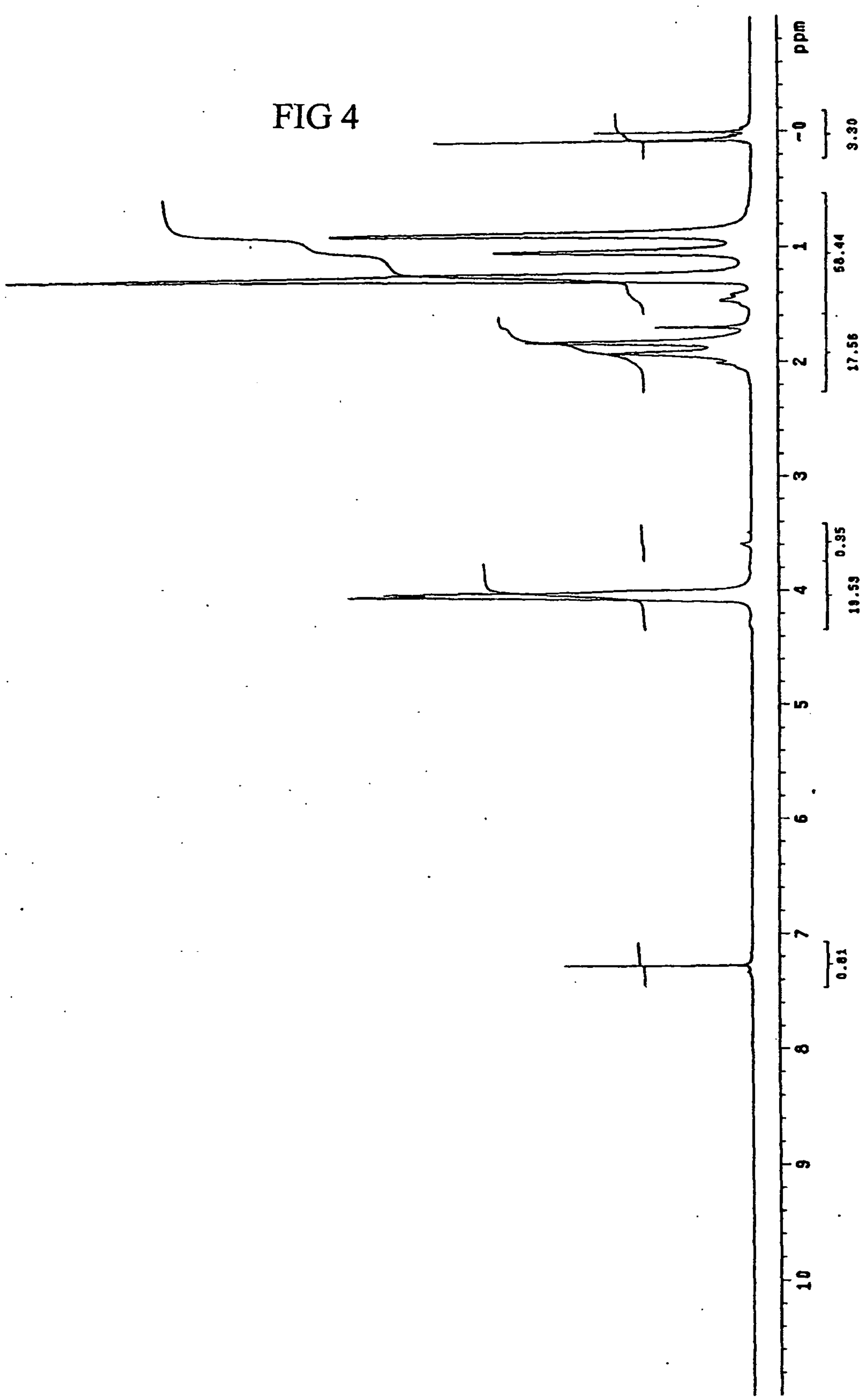

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6716441 B
- US 6630133 B
- US 20030162688 A
- JP 2006045102 A
- WO 2005049697 A
- WO 2006014328 A
- WO 2006014367 A
- US 3928644 A